# EUROPÄISCHE PATENTSCHRIFT

(11) **EP 0 534 264 B1**
(45) Veröffentlichungstag und Bekanntmachung des Hinweises auf die Patenterteilung: **13.03.1996**
(21) Anmeldenummer: 92115696.4
(22) Anmeldetag: 14.09.1992
(51) Int. Cl.: A61K 6/10

(54) **Alginat-Abformmasse**
Alginate impression material
Matériau d'empreinte à base d'alginate

(30) Priorität: 25.09.1991 DE 4131839
(43) Veröffentlichungstag der Anmeldung: 31.03.1993
(73) Patentinhaber: BAYER AG, D-51368 Leverkusen (DE)
(72) Erfinder: Winkel, Jens, Dr., W-5000 Köln 71 (DE); Voigt, Reiner, Dipl.-Ing., W-5090 Leverkusen 1 (DE); Weber, Norbert, W-5000 Köln 71 (DE)

(56) Entgegenhaltungen:
- EP-A- 0 126 824
- FR-A- 2 663 641
- GB-A- 754 375
- US-A- 4 381 947

## Beschreibung

Die Erfindung betrifft ein pastöses Zweikomponenten-Material auf Alginat-Basis, welches als Abformmaterial im Dentalbereich Anwendung findet.

Sowohl bei der Herstellung von Inlays, Kronen, Brücken und Prothesen im Zahnbereich als auch bei Korrekturen von Zahnstellungen und Kieferanomalien stellt man mit Hilfe von Abformmassen ein Negativ des interessierenden Bereichs her, das anschließend mit Modellgips ausgegossen wird. Anhand des Gipsmodells kann man dann die gewünschten Anpassungen vornehmen.

Pulverförmige Abformmassen aus Alginaten gehören zu den mit am längsten verwendeten Abformmaterialien in der zahnärztlichen Praxis (US 23 45 255). Sie bestehen zu 45 - 70 Gew.-% aus Füllstoffen (z. B. Diatomeen-, Kieselerde), 10 - 20 Gew.-% Alkali-Alginat als Polymer-Matrix, 8 - 15 Gew.-% Calciumsulfat-Dihydrat als Ca⁺⁺-Ionenspender, 0,5 - 4 Gew.-% Alkaliphosphat (Na₃PO₄, Na₄P₂O₇) als Regulator für die Verarbeitungszeit, 0,5 - 4 Gew.-% Fluoride (NaF, K₂TiF₆, K₂ZrF₆, Na₂SiF₆) zur Verbesserung der Verträglichkeit der fertigen Abformung zum Modellgips, 0,1 - 2 Gew.-% Metalloxide (MgO, ZnO) sowie geringe Mengen von Farbstoffen und Spuren von Duftstoffen.

Die vorzugsweise in Pflugscharmischem hergestellte Pulvermischung wird nach Herstellerangahen vom Zahnarzt mit Wasser (20 - 25 g Pulver: 50 ml Wasser) in einem Mischbecher mittels Spatel zu einer Paste verarbeitet, auf einen Abformlöffel plaziert und auf die abzuformende Kieferpartie gedrückt. Ca. 1 Minute 45 Sekunden nach Mischbeginn bei schnellabbindenden bzw. 3 Minuten bei normalabbindenden Alginatabformmassen verfestigt sich die Paste plötzlich zu einem festen gummiartigen Material, und der Zahnarzt kann den recht präzisen Abdruck aus dem Mund der Patienten entfernen. Um eine Dimensionsänderung dieser Negativform durch Wasserverdunstung zu vermeiden, soll der Abdruck nach 10 Minuten mit einer wässrigen Gipsaufschlämmung ausgegossen werden.

In der Verarbeitung haben Alginatpulver jedoch einige gravierende Nachteile: Neigung zu starker Staubentwicklung, Entmischung der Pulver durch unterschiedliche Rohstoffdichten, Dosierungenauigkeiten durch Pulververdichtung, umständliche Verarbeitung und Verschmutzung des Arbeitsplatzes. Die Staubneigung der Pulvermischungen kann durch Zusätze wie Polyethylen- und/oder -propylenglykol (US 43 94 172), oberflächenaktive Stoffe und Kohlenwasserstoffe wie Squalan, Squalen, Decan, Dodecan (DE 3 439 884) oder spezielle Isoparaffine (EP 0 217 270) reduziert werden.

Es hat daher nicht an Versuchen gefehlt, Alginatformulierungen zu entwickeln, die die weiteren Nachteile nicht aufweisen. So werden in der DE 31 35 567 bzw. US 4 381 947 und 4 468 484 abbindende Alginat-Zusammensetzungen beschrieben, die in Form eines pastösen Zweikomponenten-Systems aus dem Komponenten A und B bestehen und nach dem Vermischen dieser abbinden.

Die Komponente A kann lt. Beispiele 1 - 4 enthalten:
- 11 - 12 Gew.-Teile: Natrium- oder Kaliumalginat
- 50 - 84 Gew.-Teile: Diatomeenerde
- 0,5 - 2 Gew.-Teile: K₄P₂O₇ oder Na₄P₂O₇
- 0 - 4 Gew.-Teile: Diethylenglykol oder Propylenglykol
- 0 - 5 Gew.-Teile: Dextrose oder Sorbit
- 150 Gew.-Teile: Wasser
- Spuren: Konservierungsmittel
- Spuren: Geschmacksstoffe
Die Komponente B kann lt. Beispiele 1 - 4 und 6 a - f bestehen aus:
- 0 - 10 Gew.-Teile: MgO, ZnO
- 10 - 60 Gew.-Teile: CaSO₄ x 2 H₂O, ZnSO₄
- 2 - 4 Gew.-Teile: K₄P₂O₇ oder Na₄P₂O₇
- 35 - 60 Gew.-Teile: Glycerin, Propylenglykol, Diethylenglykol, Mineralöl oder Oleylalkohol
- 0 - 5 Gew.-Teile: Silikonöl
- 0 - 22 Gew.-Teile: Diatomeenerde, Siliciumdioxid, Talk
- 0 - 2 Gew.-Teile: NaF, ZnF₂
Mit diesen Pasten besteht zwar nicht mehr wie bei den Alginat-Pulvern das Problem der Entmischung und des Stäubens jedoch kommt es bei Lagerung zu einer drastischen Verschlechterung der Produkteigenschaften. So wurde in DE 31 35 567 angegeben, daß nach Vermischen der soeben hergestellten Komponente A und der soeben hergestellten Komponente B im Volumen-Verhältnis 4:1 nach dem Abbinden die vernetzte Masse eine Druckfestigkeit von 900 g/6,45 cm² (= 0,14 kp/cm² = 0,014 N/mm²) aufweist. Bereits 30 Tage nach Herstellung der Komponenten ist die Druckfestigkeit der daraus hergestellten vernetzten Masse auf 600 g/6,45 cm² (= 0,09 kp/cm² = 0,009 N/mm²) abgesunken. Diese mangelhafte Lagerstabilität hat auch dazu beigetragen, daß bisher pastenförmige Alginat-Massen keine wesentlichen Anteile am Alginatmarkt erringen konnten.

Gegenstand der Erfindung sind Abformmassen auf Alginat-Basis, bestehend aus zwei Pastenkomponenten, dadurch gekennzeichnet, daß sich die in Gegenwart von Wasser anwendungstechnisch nachteilig verändernden Bestandteile in der wasserfreien Paste A und sich die in Gegenwart von Wasser sich anwendungstechnisch nicht nachteilig verändernden Bestandteile und Wasser in der Paste B befinden, also lagerstabile pastöse 2 Komponenten-Abformmaterialien auf Alginat-Basis, in denen Bestandteile wie Alkalialginat, Calciumsulfat-Dihydrat, Alkaliphosphat, Fluoride und Metalloxide in einer Paste A und inerte Füllstoffe in einer Paste B eingebracht werden. Grundlagen für die Paste A sind Glyzerin, Glykole, Polyethylen- und -propylenglykole sowie Mischungen dieser in übrigen wasserfreien Substanzen. In der auf Basis von Wasser hergestellten Paste B werden Gelbildner eingesetzt, um einerseits die Sedimentation der Füllstoffe zu verhindern und zum anderen, damit durch Einstellung der Viskosität auf die der Paste A ein späteres leichtes Vermischen mit derselben gewährleistet ist.

Solche Gelbildner sind z.B. Methylcellulose, Hydroxypropylcellulose, Hydroxyethylcellulose, Carboxymethylcellulose und Alkalipolyacrylate sowie -copolymerisate.

Die so erhaltenen Pasten haben hervorragende verarbeitungstechnische Eigenschaften und sind lagerstabil.

Erfindungsgemäße Abformmassen sind dadurch gekennzeichnet, daß die Paste A
- a) 10 - 25 Gew.-%: Calciumsulfat-Dihydrat
- b) 15 - 23 Gew.-%: Kalium- und/oder Natriumalginat
- c) 3 - 7 Gew.-%: Metalloxid (MgO, ZnO)
- d) 0,5 - 3 Gew.-%: Alkaliphosphat (Na₄P₂O₇, Na₃PO₄, K₄P₂O₇)
- e) 0,5 - 2 Gew.-%: Farbpigment sowie
- f) 50 - 65 Gew.-%: Pastenbildner
und daß die Paste B
- g) 75 - 85 Gew.-%: entmineralisiertes Wasser
- h) 15 - 25 Gew.-%: Diatomeen- oder Kieselerde
- i) Spuren: Duftstoffe sowie
- j) 0,3 - 1 Gew.-%: Gelbildner
enthalten. Nach dem Vermischen der Paste A und der Paste B im Gewichts- und/oder Volumenverhältnis von 1:3,5 bis 1:10 erhalt man eine gebrauchsfähige Abformmasse, die innerhalb von 1 Min. 15 Sek. bis 3 Min. zu einer festen gummiartigen Masse abbindet.

Die Einsatzstoffe a) - e) und h) -i) sind allgemein bekannt und in vielen Lehrbüchern und Patentdokumenten beschrieben.

Bei dem Pastenbildner f) handelt es sich um Glyzerin, Glykole, Polyethylen-und Polypropylenglykole. Bevorzugt werden Polyethylenglykole mit mittleren Molekulargewichten von 300 bis 4000 und besonders bevorzugt Mischungen von diesen.

Gelbildner j) sind Carboxymethyl-, Methyl-, Hydroxyethyl- und Hydroxypropylcellulose sowie Alkalipolyacrylate. Bevorzugt werden Alkalisalze der Polyacrylsäure und Polyacrylamidpolyacrylsäure und besonders bevorzugt Natriumsalze von

Acrylsäure-Acrylamid-Copolymerisaten, wie ® HOSTACERIN PN 73 (Fa. Hoechst).

Die erfindungsgemäßen Abformmassen zeichnen sich durch eine gute Lagerstabilität aus, das Alkali-Alginat in der Paste A erleidet keinen Molekülabbau. Durch Variation der Anteile an Alkaliphosphat kann gezielt die gewünschte spätere Abbindezeit eingestellt werden, bevor die pulverförmigen Anteile mit Polyethylenglykol zu der Paste A verarbeitet werden. Der Gelbildner in der Paste B verhindert die Sedimentation der Füllstoffe und bringt die Paste in den Viskositätsbereich von dem der Paste A.

Damit ist eine gute Mischbarkeit beider Pasten bei Anwendung gegeben. Die erfindungsgemäßen Abformmassen erfüllen die Anforderungen der Spezifikationen ADA 18 und ISO 1563-2 für Alginat-Abformmassen.

Das nachfolgende Beispiel, in dem alle Teile Gewichtsteile bedeuten, erläutert die Erfindung:
Paste A:
14 Teile Calciumsulfat-Dihydrat, 5 Teile Magnesiumoxid, 19 Teile Natriumalginat, 1,5 Teile Tetranatriumpyrophosphat, 3,5 Teile Kaliumfluorotitanat und 1 Teil Farbpigment werden in einer Kugelmühle miteinander vermahlen. 40 Teile Polyethylenglykol mit einem mittleren Molgewicht von 400 werden in einen Planetenmischer vorgelegt und mit der oben genannten Pulvermischung zu einer homogenen Paste vermischt. Anschließend werden 16,5 Teile des Polyethylenglykols hinzugegeben und eingearbeitet. Die Herstellung der Paste erfolgt unter Ausschluß von Feuchtigkeit.

Paste B: 0,5 Teile ® HOSTACERIN PN 73 (Hoechst) und 2,5 Teile Diatomeenerde werden vorgemischt. Diese Vormischung wird mit weiteren 18 Teilen Diatomeenerde in 79 Teile entmineralisiertem Wasser zu einer homogenen Paste verrührt.

Die Paste A wurde mit Paste B im Gewichts-Verhältnis 1:4,5 gemischt. Die Prüfung erfolgte nach der Spezifikation ISO 1563-2.

| | | |
|---|---|---|
| Prüfung | sofort nach Herstellung der Pasten | 90 Tage nach Herstellung der Pasten |
| Abbindezeit | 1 Min. 30 Sec. | 1 Min. 35 Sec. |
| Bleib. Deformation | 2,8 % | 3,1 % |
| Druckfestigkeit | 0,82 N/mm² | 0,76 N/mm² |

## Patentansprüche

1. Abformmassen auf Alginat-Basis, bestehend aus zwei Pastenkomponenten, dadurch gekennzeichnet, daß die Paste A
a) 10 - 25 Gew.-% Calciumsulfat-Dihydrat
b) 15 - 23 Gew.-% Kalium- und/oder Natriumalginat
c) 3 - 7 Gew.-% Metalloxid (MgO, ZnO)
d) 0,5 - 3 Gew.-% Alkaliphosphat (Na₄P₂O₇, Na₃PO₄, K₄P₂O₇)
e) 0,5 - 2 Gew.-% Farbpigment sowie
f) 50 - 65 Gew.-% Pastenbildner
und die Paste B
g) 75 - 85 Gew.-% entmineralisiertes Wasser
h) 15 - 25 Gew.-% Diatomeen- oder Kieselerde
i) Spuren Duftstoffe sowie
j) 0,3 - 1 Gew.-% Gelbildner
enthalten.

2. Abformmassen nach Anspruch 1 , dadurch gekennzeichnet, daß der Pastenbildner f) in Paste A Glyzerin, Glykole, Polyethylen- sowie Polypropylenglykole und der Gelbildner j) in Paste B Carboxymethyl-, Methyl-, Hydroxyethyl- und Hydroxypropylcellulose sowie Alkalisalze von Polyacrylsäure und -copolymerisaten sind.

3. Abformmassen nach den Ansprüchen 1 und 2, dadurch gekennzeichnet, daß als Pastenbildner f) in Paste A Polyethylenglykole mit mittleren Molekulargewichten von 300 bis 4000 und als Gelbildner j) in Paste B Alkalisalze der Polyacrylsäure und Polyacrylamidpolyacrylsäure verwendet werden.

4. Abformmassen nach den Ansprüchen 1 - 3, dadurch gekennzeichnet, daß die Paste A und die Paste B im Volumen- oder Gewichtsverhältnis von 1:3,5 bis 1:10 zu einer Dentalabdruckmasse durch Vermischung verarbeitet werden können.

5. Verwendung der Abformmassen nach den Ansprüchen 1 - 4 zur Herstellung von Abdrücken im Dentalbereich.

## Claims

1. Alginate-based impression compositions consisting of two paste components, characterised in that paste A comprises
a) 10 - 25 % by weight of calcium sulphate dihydrate
b) 15 - 23 % by weight of potassium alginate and/or sodium alginate
c) 3 - 7 % by weight of metal oxide (MgO, ZnO)
d) 0.5 - 3 % by weight of alkali metal phosphate (Na₄P₂O₇, Na₃PO₄, K₄P₂O₇)
e) 0.5 - 2 % by weight of coloured pigment and
f) 50 - 65 % by weight of paste-forming agent
and paste B comprises
g) 75 - 85 % by weight of demineralised water
h) 15 - 25 % by weight of diatomaceous earth or silica
i) traces of fragrances and
j) 0.3 - 1 % by weight of gel-forming agent.

2. Impression compositions according to Claim 1, characterised in that the paste-forming agents f) in paste A are glycerol, glycols, polyethylene glycols and polypropylene glycols and the gel-forming agents j) in paste B are carboxymethyl-, methyl-, hydroxyethyl- and hydroxypropylcellulose and alkali metal salts of polyacrylic acid and copolymers.

3. Impression compositions according to Claims 1 and 2, characterised in that polyethylene glycols having average molecular weights of 300 to 4000 are used as the paste-forming agents f) in paste A and alkali metal salts of polyacrylic acid and polyacrylamidepolyacrylic acid are used as the gel-forming agents j) in paste B.

4. Impression compositions according to Claims 1 - 3, characterised in that paste A and paste B can be processed by mixing in a volume or weight ratio of 1:3.5 to 1:10 to give a dental impression composition.

5. Use of the impression compositions according to Claims 1 - 4 for the production of impressions in the dental sector.

## Revendications

1. Compositions pour empreintes à base d'alginate, constituée de deux composants en pâte, caractérisées en ce que la pâte A contient :
a) 10 à 25 % en poids de sulfate de calcium dihydraté
b) 15 à 23 % en poids d'alginate de potassium et/ou de sodium
c) 3 à 7 % en poids d'oxyde métallique (MgO, ZnO)
d) 0,5 à 3 % en poids de phosphate alcalin (Na₄P₂O₇, Na₃PO₄, K₄P₂O₇)
e) 0,5 à 2 % en poids de pigment de couleur ainsi que
f) 50 à 65 % en poids d'agent de mise en pâte
et la pâte B contient
g) 75 à 85 % en poids d'eau déminéralisée
h) 15 à 25 % en poids de terre de diatomées ou de silice
i) des traces d'arômes ainsi que
j) 0,3 à 1 % en poids d'agent gélifiant

2. Compositions pour empreintes suivant la revendication 1, caractérisées en ce que l'agent de mise en pâte f) présent dans la pâte A consiste en glycérol, glycols, polyéthylène- ainsi que polypropylèneglycols et l'agent gélifiant j) dans la pâte B consiste en carboxyméthylcellulose, méthylcellulose, hydroxyéthylcellulose, hydroxypropylcellulose ainsi qu'en sels alcalins de polymère d'acide acrylique et de copolymère d'acrylamide et d'acide acrylique.

3. Compositions pour empreintes suivant les revendications 1 et 2, caractérisées en ce qu'on utilise comme agent de mise en pâte f) dans la pâte A des polyéthylèneglycols ayant des poids moléculaires moyens de 300 à 4000 et comme gélifiant j) dans la pâte B des sels alcalins de polymère d'acide acrylique et de copolymère d'acrylamide et d'acide acrylique.

4. Compositions pour empreintes suivant les revendications 1 à 3, caractérisées en ce que la pâte A et la pâte B peuvent être transformées par mélange en une pâte pour la prise d'empreintes dentaires dans le rapport en volume ou en poids de 1:3,5 à 1:10.

5. Utilisation des compositions pour empreintes suivant les revendications 1 à 4 pour la prise d'empreintes dans le domaine de l'art dentaire.
